# EUROPEAN PATENT APPLICATION

(11) **EP 3 469 995 A1**
(43) Date of publication of application: **17.04.2019**
(21) Application number: 17802238.0
(22) Date of filing: 26.06.2017
(51) Int. Cl.: A61B 17/072

(54) **SURGICAL STAPLER**

(30) Priority: 23.05.2016 CN 201610345074
(71) Applicant: Shanghai Yisi Medical Technology Co., Ltd., Shanghai 2013184 (CN); Yisi Suzhou Medical Technology Co., Ltd., Suzhou City, Jiangsu 215163 (CN)
(72) Inventor: NIE, Honglin, Shanghai 201318 (CN); YANG, Guang, Shanghai 201318 (CN); ZHANG, Xiliang, Shanghai 201318 (CN); SHI, Xiufeng, Shanghai 201318 (CN); LI, Anhua, Shanghai 201318 (CN); BAO, Minghui, Shanghai 201318 (CN); JIANG, Yang, Shanghai 201318 (CN)
(74) Representative: Mansfield, Peter Turquand
(86) International application number: PCT/CN2017/089939
(87) International publication number: WO 2017/202392

(57) **Abstract**

The present invention discloses a surgical stapler. The surgical stapler includes a first handle part, a second handle part, and a adjustment mechanism; the first handle part includes a staple cartridge component and a drive assembly, and the second handle part includes a staple abutting seat; a locking mechanism is arranged on the first handle part or/and the second handle part; the first handle part and the second handle part may rotate via the rotation shaft, and lock in a longitudinal direction; the adjustment mechanism is located between the first handle part and the second handle part; the adjustment mechanism may make, by means of adjustment, the second handle part rotate relative to the first handle part via the rotation shaft thereon. The surgical stapler provided by the present invention may make, by means of the adjustment mechanism at the proximal end of the handle, the thickness of the tissue after clamping reach the same at the proximal and distal ends, so as to improve the formation rate of the suturing staple, and reduce the bleeding of the suturing staple, anastomotic leakage and the like.

## Description

### Technical Field

The present invention belongs to the technical field of medical apparatus and instruments, and relates to a stapler structure, and in particular, relates to a surgical stapler.

### Background

The function and principle of a surgical stapler is to clamp tissues by closing two corresponding handle components (generally including a staple abutting seat component and a staple cartridge component), and the suturing staple in the staple cartridge component of the stapler is then extruded and the tissue is stitched together. In some staplers, there is also a cutting knife for cutting the stitched tissue.

The surgical stapler with the above function includes a first handle part and a second handle part. The first handle part includes a dismountable staple cartridge component, a locking rod, and a drive assembly. The staple cartridge component generally includes a staple cartridge, several suturing staples, and several staple pushing blocks. There are multiple rows of holes on the staple cartridge, suturing staples are inside the holes, and the upper surface of the staple cartridge is a tissue contact surface. The second handle part includes a staple abutting seat, and the staple abutting seat includes a staple forming surface, and the staple forming surface includes multiple rows of staple forming grooves.

The first handle part and the second handle part may be connected and locked by means of the locking rod located on the first handle part to bring together tissues that need to be stitched.

The drive assembly includes a wedge pushing element. When the drive assembly moves from the proximal end of the stapler to the distal end, the drive assembly drives the suturing staple in the staple cartridge to form in the staple forming groove of the staple abutting seat. Generally, the drive assembly also includes a cutting knife used for cutting tissues in the middle of the multiple rows of staples after the tissue is sutured by suturing staple.

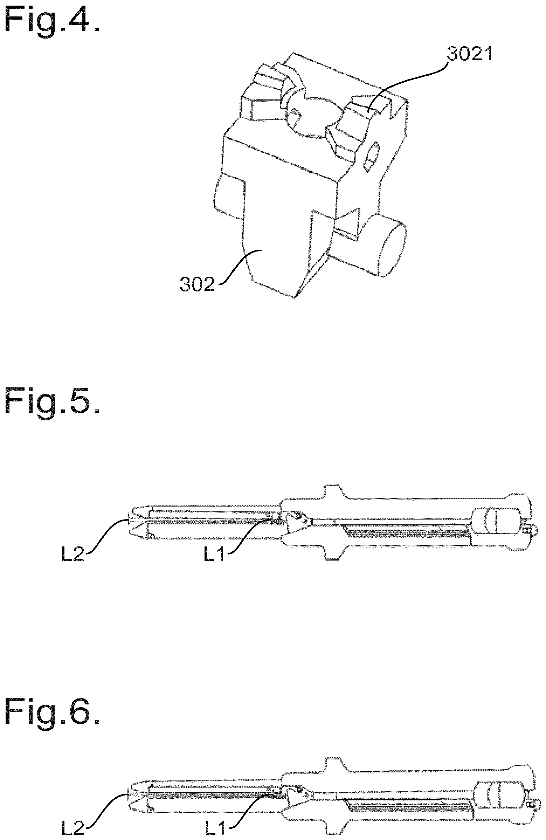

In an existing stapler design, when different tissues are encountered, because the tissue toughness, thickness, etc. are inconsistent, when the stapler brings together the thick tissues, the compression thickness of the proximal and distal ends of the clamped tissue may be inconsistent. Generally, the compression thickness generated at distal end is greater than that of the proximal end, as shown in FIG. 5, and at this time, L2>L1. In this case, the heights of the suturing staples at the proximal and distal ends are inconsistent, the formed staple at the distal end is too high and even the forming is poor, leading to bleeding of the suturing staple, anastomotic leakage and the like.

On such basis, a new stapler is desired to be designed to overcome the above defects of the existing stapler.

### Summary

A technical problem to be solved by the present invention is to provide a surgical stapler which may make the thickness of the tissue after clamping reach the same at the proximal and distal ends, so as to improve the formation rate of the suturing staple, and reduce the bleeding of the suturing staple, anastomotic leakage and the like.

To solve the above technical problem, the following technical solutions are used in the present invention:
A surgical stapler, where the surgical stapler includes a first handle part, a second handle part, and an adjustment mechanism;
the first handle part includes a staple cartridge base, a dismountable staple cartridge component, a locking rod, and a drive assembly, and the second handle part includes a staple abutting seat and a rotation shaft;
the first handle part and the second handle part are assembled together, and are locked by the locking rod on the first handle part in a longitudinal direction;
the adjustment mechanism is placed on a proximal end of the stapler and is located between the first handle part and the second handle part; the adjustment mechanism is rotated to make the second handle part rotate relative to the first handle part about the rotation shaft thereon, so that the thickness of the tissue after clamping reaches the same value at the proximal and distal ends;
the adjustment mechanism is a cam mechanism and includes a rotary dial, a rotary dial button, a pin, a compression spring, an adjustment slider, and a sliding pin; the rotary dial is connected to the first handle part via the pin and the compression spring; the adjustment slider is connected to the second handle part via the sliding pin; a circular hole is arranged on the staple cartridge base, and is coaxial with the pin, the rotary dial, and the adjustment slider; and
the rotary dial button of the adjustment mechanism is connected to the rotary dial, the rotary dial comprises at least two stepped surfaces, and the adjustment slider is engaged with any of the stepped surfaces of the rotary dial; when the rotary dial button is rotated, the rotary dial also rotates, so that the adjustment slider engaged with the rotary dial generates displacement orthogonal to the stepped surface, so as to drive the second handle part via the sliding pin to rotate relative to the first handle part, until the thickness of the tissue after clamping reaches the same value at the proximal and distal ends.

A surgical stapler, where the surgical stapler includes a first handle part, a second handle part, and an adjustment mechanism;
the first handle part includes a staple cartridge component and a drive assembly, and the second handle part includes a staple abutting seat; a locking mechanism is arranged on the first handle part or/and the second handle part, and the first handle part and the second handle part may rotate via a rotation mechanism and lock via the locking mechanism in a longitudinal direction; and
the adjustment mechanism is located between the first handle part and the second handle part; the adjustment mechanism is adjusted to make the second handle part rotate relative to the first handle part via the rotation mechanism thereon.

As an optimal solution of the present invention, the adjustment mechanism is disposed on a proximal end of the stapler; the adjustment mechanism adjusts its height so that the second handle part rotates relative to the first handle part, and the thickness of the tissue after clamping by the stapler reaches the same value at the proximal and distal ends.

As an optimal solution of the present invention, the adjustment mechanism is a cam mechanism.

As an optimal solution of the present invention, the cam mechanism includes a rotary dial button, a rotary dial, and an adjustment slider, where the rotary dial button is connected to the rotary dial; the rotary dial comprises at least two stepped surfaces, one end of the adjustment slider is engaged with any of the stepped surfaces of the rotary dial, and the other end is connected to the second handle part via a sliding mechanism; when the rotary dial button is rotated, the rotary dial rotates along with the button, so that the adjustment slider engaged with the dial generates displacement orthogonal to the stepped surface, so as to drive the second handle part via the sliding mechanism to rotate relative to the first handle part, until the thickness of the tissue after clamping reaches the same value at the proximal and distal ends.

As an optimal solution of the present invention, the adjustment mechanism includes a rotary dial, a rotary dial button, a pin, a compression spring, an adjustment slider, and a sliding pin; the rotary dial is connected to the first handle part via the pin and the compression spring, and the adjustment slider is connected to the second handle part via the sliding pin; a circular hole is arranged on the staple cartridge base, and is coaxial with the pin, the rotary dial, and the adjustment slider; and
the rotary dial button of the adjustment mechanism is connected to the rotary dial, the rotary dial includes at least two stepped surfaces, and the adjustment slider is engaged with any of the stepped surfaces of the rotary dial; when the rotary dial button is rotated, the rotary dial also rotates, so that the adjustment slider engaged with the rotary dial generates displacement orthogonal to the stepped surface, so as to drive the second handle part via the sliding pin to rotate relative to the first handle part, until the thickness of the tissue after clamping reaches the same value at the proximal and distal ends.

As an optimal solution of the present invention, the adjustment mechanism includes a wedge slider, and the wedge slider has a sliding slope; the sliding slope is a non-horizontal surface; the wedge slider is arranged above the staple cartridge base, and the sliding slope fits against the staple abutting seat; the sliding slope has a push button; the push button is pushed to make the wedge slider locate in different locations, so that the staple abutting seat moves depending on the location of the sliding slope, and further the proximal end of the staple abutting seat rises/lowers and the distal end lowers/rises, until the thickness of the tissue after clamping reaches the same value at the proximal and distal ends.

As an optimal solution of the present invention, the adjustment mechanism includes a threaded adjustment screw, the thread of the adjustment screw fits a thread hole located on the first handle part/the second handle part, and the screw top fits the second handle part/the first handle part.

Beneficial effects of the present invention are that: the surgical stapler provided by the present invention may, by means of the adjustment mechanism at the proximal end of the handle, make the thickness of the tissue after clamping reach the same value at the proximal and distal ends, so as to improve the formation rate of the suturing staple, and reduce bleeding of the suturing staple, anastomotic leakage and the like.

### Description of Accompany Drawings

FIG. 1 is a schematic structural diagram of a surgical stapler of the present invention in embodiment 1;
FIG. 2 is a schematic structural diagram of an adjustment mechanism in the surgical stapler of the present invention in embodiment 1;
FIG. 3 is a schematic structural diagram of a rotary dial in the surgical stapler of the present invention in embodiment 1;
FIG. 4 is a schematic structural diagram of an adjustment slider in the surgical stapler of the present invention in embodiment 1;
FIG. 5 is a schematic diagram of the surgical stapler before adjustment in embodiment 1;
FIG. 6 is a schematic diagram of the surgical stapler after adjustment in embodiment 1;
FIG. 7 is a schematic diagram of an adjustment mechanism in the surgical stapler of the present invention in embodiment 2;
FIG. 8 is a schematic diagram of a wedge slider in the surgical stapler of the present invention in embodiment 2;
FIG. 9 is a schematic diagram of a surgical stapler of the present invention in embodiment 3; and
FIG. 10 is a schematic diagram of an adjustment screw in the surgical stapler of the present invention in embodiment 3.

### Embodiments

Preferred embodiments of the present invention are described in detail in combination with the accompanying drawings.

### Embodiment 1

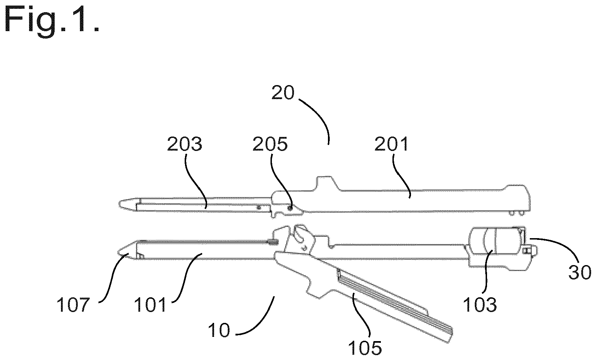

Referring to FIGs. 1 to 6, the present invention discloses a surgical stapler, and the surgical stapler includes a first handle part 10, a second handle part 20, and an adjustment mechanism 30.

In this embodiment, the first handle part 10 includes a staple cartridge base 101, a dismountable staple cartridge component (including a staple cartridge 107, several staple pushing blocks, and several suturing staples), a locking rod 105, a drive assembly (a pushing button 103, a pushing sheet, and a cutting knife in this embodiment); the locking rod 105 is rotatably installed on the first handle part 10. The second handle part 20 includes a staple abutting seat housing 201, a staple abutting seat 203, and a rotation shaft 205 (or other rotation mechanism), and the staple abutting seat 203 has a staple forming surface.

The first handle part 10 and the second handle part 20 are assembled together, and are locked via the locking rod 105 on the first handle part 10 in a longitudinal direction.

The adjustment mechanism 30 is placed on a proximal end of the stapler and is located between the first handle part 10 and the second handle part 20 (more specifically, between the staple cartridge base 101 and the staple abutting seat 203); the adjustment mechanism 30 is rotated to make the second handle part 20 rotate relative to the first handle part 10 about the rotation shaft 205 thereon, so that the thickness of the tissue after clamping reaches the same value at the proximal and distal ends.

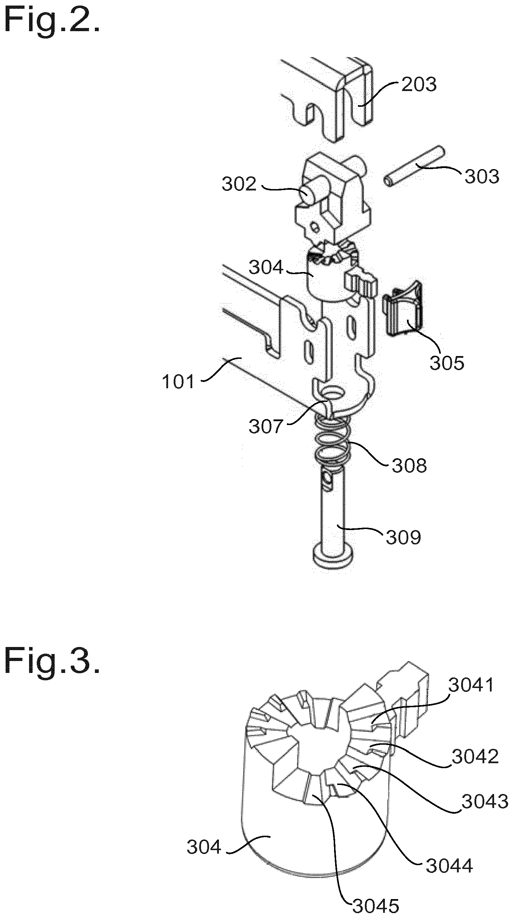

Referring to FIG. 2, in this embodiment, the adjustment mechanism 30 is a cam mechanism. The cam mechanism includes a rotary dial button 305, a rotary dial 304, a pin 309, a compression spring 308, an adjustment slider 302, a sliding pin 303 (or any other sliding mechanism).

The rotary dial 304 is connected to the first handle part 10 via the pin 309 and the compression spring 308; the adjustment slider 302 is connected to the second handle part 20 via the sliding pin 303; a circular hole is arranged on the staple cartridge base 101, and is coaxial with the pin 309, the rotary dial 304, and the adjustment slider 302, as shown in FIG. 2.

As shown in FIG. 3 and FIG. 4, the rotary dial button 305 is connected to the rotary dial 304, and the rotary dial 304 includes at least two stepped surfaces 3041, 3042, 3043, 3044, and 3045 (as shown in FIG. 3). One end of the adjustment slider 302 is engaged with any of the stepped surfaces of the rotary dial 304, and the other end, held by the sliding pin 303, bears against the second handle part 20. When the rotary dial button 305 is rotated, the rotary dial 304 also rotates, so that the adjustment slider 302 (including a second stepped surface 3021 fitting the stepped surface of the rotary dial 304) engaged with the rotary dial 304 generates displacement orthogonal to the stepped surface via the sliding pin 303, so as to drive the second handle part 20 to rotate relative to the first handle part 10, until the thickness of the tissue after clamping reaches the same value at the proximal and distal ends, as shown in FIG. 6, and at this time, L1≈L2.

Certainly, the adjustment mechanism may use other methods to make the second handle part rotate relative to the first handle part, so that the thickness of the tissue after clamping reach the same at the proximal and distal ends.

### Embodiment 2

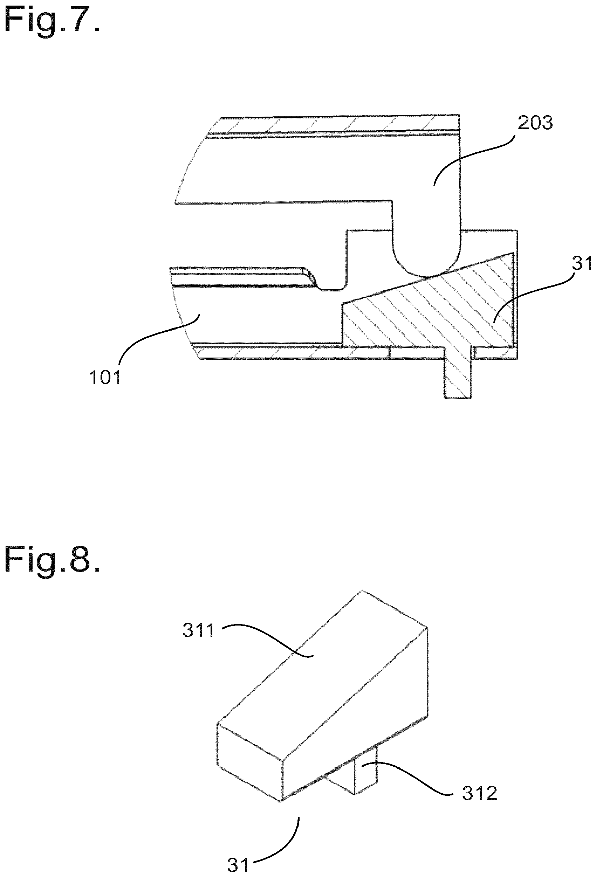

This embodiment differs from embodiment 1 in that in this embodiment, referring to FIGs. 7 and 8, the adjustment mechanism includes a wedge slider 31, the wedge slider 31 has a sliding slope 311, and the sliding slope 311 is a non-horizontal surface. The wedge slider 31 is placed above the staple cartridge base 101, and the sliding slope 311 fits against the staple abutting seat 203. The sliding slope 311 has a push button 312. The push button 312 may make the wedge slider 31 locate in different locations, so that the staple abutting seat 203 moves depending on the location of the sliding slope 311, and further makes the proximal end of the staple abutting seat 203 rise (or lower) and the distal end lower (or rise), until the thickness of the tissue after clamping reaches the same value at the proximal and distal ends, as shown in FIG. 6, at this time, L1≈L2.

### Embodiment 3

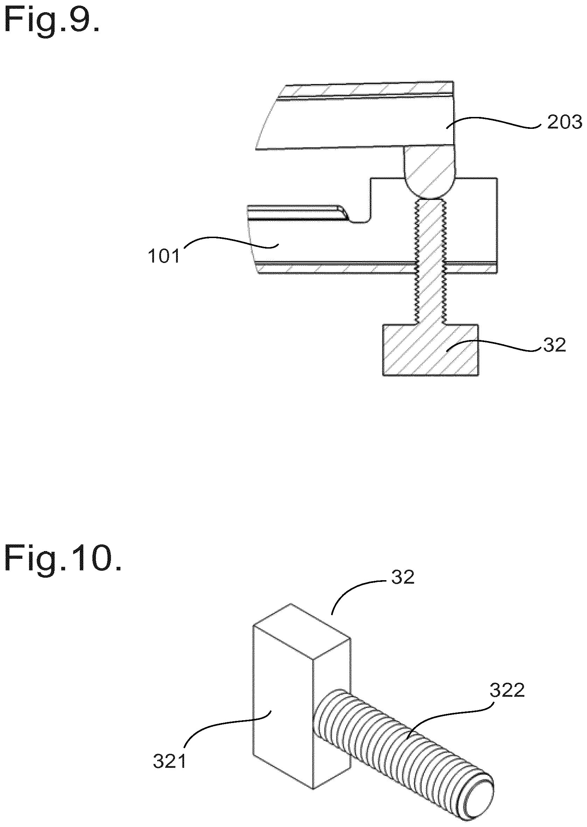

This embodiment differs from embodiment 1 in that in this embodiment, referring to FIGs. 9 and 10, the adjustment mechanism includes a threaded adjustment screw 32 (including an adjustment knob 321 and a threaded 322), the thread of the adjustment screw 32 fits a threaded hole located on the staple cartridge base 101, and the screw top fits the staple abutting seat 203.

When the adjustment knob 321 on the adjustment screw is rotated manually, the relative position of the top of the adjustment screw 32 changes relative to the staple cartridge base, so that the proximal end of the staple abutting seat 203 rises (or lowers) and the distal end lowers (or rises), until the thickness of the tissue after clamping reaches the same value at the proximal and distal ends, as shown in FIG. 6, L1≈L2.

### Embodiment 4

A surgical stapler, where the surgical stapler includes a first handle part, a second handle part, and an adjustment mechanism. The first handle part includes a staple cartridge component and a drive assembly. The second handle part includes a staple abutting seat; a locking mechanism is disposed on the first handle part or/and the second handle part, and the first handle part and the second handle part may rotate about the rotation shaft (or any other rotation mechanism) and is locked by the locking mechanism in a longitudinal direction. The adjustment mechanism is located between the first handle part and the second handle part. The adjustment mechanism is adjusted to make the second handle part rotate, about the rotation shaft thereon, relative to the first handle part.

In conclusion, the surgical stapler provided by the present invention may, by means of the adjustment mechanism at the proximal end of the handle, make the thickness of the tissue after clamping reach the same value at the proximal and distal ends, so as to improve the formation rate of the suturing staple, and reduce the bleeding of the suturing staple, anastomotic leakage and the like.

The description and application of the present invention are intended to be illustrative, and not intended to limit the scope of the invention. Variations and modifications of the embodiments disclosed herein are possible, and various alternative and equivalent components of the embodiments are well known to those of ordinary skill in the art. It is apparent to those skilled in the art that the present invention may be embodied in other forms, structures, arrangements, ratios, and other components, materials and components without departing from the spirit or essential characteristics of the invention. Other variations and modifications of the embodiments disclosed herein may be made without departing from the scope and spirit of the invention.

## Claims

1. A surgical stapler, wherein the surgical stapler comprises a first handle part, a second handle part, and an adjustment mechanism;
the first handle part comprises a staple cartridge base, a dismountable staple cartridge component, a locking rod, and a drive assembly, and the second handle part comprises a staple abutting seat and a rotation shaft;
the first handle part and the second handle part are assembled together, and are locked via the locking rod on the first handle part in a longitudinal direction;
the adjustment mechanism is placed on a proximal end of the stapler and is located between the first handle part and the second handle part; the adjustment mechanism is rotated to make the second handle part rotate relative to the first handle part about the rotation shaft thereon, so that the thickness of the tissue after clamping reaches the same value at the proximal and distal ends;
the adjustment mechanism is a cam mechanism and comprises a rotary dial, a rotary dial button, a pin, a compression spring, an adjustment slider, and a sliding pin; the rotary dial is connected to the first handle part via the pin and the compression spring; the adjustment slider is connected to the second handle part via the sliding pin; a circular hole is arranged on the staple cartridge base, and is coaxial with the pin, the rotary dial, and the adjustment slider; and
the rotary dial button of the adjustment mechanism is connected to the rotary dial, the rotary dial comprises at least two stepped surfaces, and the adjustment slider is engaged with any of the stepped surfaces of the rotary dial; when the rotary dial button is rotated, the rotary dial also rotates, so that the adjustment slider engaged with the rotary dial generates displacement orthogonal to the stepped surface, so as to drive the second handle part via the sliding pin to rotate relative to the first handle part, until the thickness of the tissue after clamping reaches the same value at the proximal and distal ends.

2. A surgical stapler, wherein the surgical stapler comprises a first handle part, a second handle part, and an adjustment mechanism;
the first handle part comprises a staple cartridge component and a drive assembly, and the second handle part comprises a staple abutting seat; a locking mechanism is arranged on the first handle part or/and the second handle part, and the first handle part and the second handle part may rotate via a rotation mechanism and lock via the locking mechanism in a longitudinal direction; and
the adjustment mechanism is located between the first handle part and the second handle part; the adjustment mechanism is adjusted to make the second handle part rotate relative to the first handle part via the rotation mechanism thereon.

3. The surgical stapler according to claim 2, wherein:
the adjustment mechanism is disposed on a proximal end of the stapler; the adjustment mechanism adjusts its height so that the second handle part rotates relative to the first handle part, and the thickness of the tissue after clamping by the stapler reaches the same value at the proximal and distal ends.

4. The surgical stapler according to claim 2, wherein:
the adjustment mechanism is a cam mechanism.

5. The surgical stapler according to claim 4, wherein:
the cam mechanism comprises a rotary dial button, a rotary dial, and an adjustment slider, wherein the rotary dial button is connected to the rotary dial; the rotary dial comprises at least two stepped surfaces, one end of the adjustment slider is engaged with any of the stepped surfaces of the rotary dial , and the other end is connected to the second handle part via a sliding mechanism; when the rotary dial button is rotated, the rotary dial rotates along with the button, so that the adjustment slider engaged with the dial generates displacement orthogonal to the stepped surface, so as to drive the second handle part via the sliding mechanism to rotate relative to the first handle part, until the thickness of the tissue after clamping reaches the same value at the proximal and distal ends.

6. The surgical stapler according to claim 2, wherein:
the adjustment mechanism comprises a rotary dial, a rotary dial button, a pin, a compression spring, an adjustment slider, and a sliding pin; the rotary dial is connected to the first handle part via the pin and the compression spring, and the adjustment slider is connected to the second handle part via the sliding pin; a circular hole is arranged on the staple cartridge base, and is coaxial with the pin,
the rotary dial, and the adjustment slider; and
the rotary dial button of the adjustment mechanism is connected to the rotary dial,
the rotary dial comprises at least two stepped surfaces, and the adjustment slider is engaged with any of the stepped surfaces of the rotary dial; when the rotary dial button is rotated, the rotary dial also rotates, so that the adjustment slider engaged with the rotary dial generates displacement orthogonal to the stepped surface, so as to drive the second handle part via the sliding pin to rotate relative to the first handle part, until the thickness of the tissue after clamping reaches the same value at the proximal and distal ends.

7. The surgical stapler according to claim 2, wherein:
the adjustment mechanism comprises a wedge slider, and the wedge slider has a sliding slope; the sliding slope is a non-horizontal surface; the wedge slider is arranged above the staple cartridge base, and the sliding slope is fit with the staple abutting seat; the sliding slope has a push button; the push button is pushed to make the wedge slider locate in different locations, so that the staple abutting seat moves depending on the location of the sliding slope, and further the proximal end of the staple abutting seat rises/lowers and the distal end lowers/rises, until the thickness of the tissue after clamping reaches the same value at the proximal and distal ends.

8. The surgical stapler according to claim 2, wherein:
the adjustment mechanism comprises a threaded adjustment screw, the thread of the adjustment screw fits a thread hole located on the first handle part/the second handle part, and the screw top fits against the second handle part/the first handle part.
